# EUROPEAN PATENT SPECIFICATION

(11) **EP 2 373 357 B1**
(45) Date of publication and mention of the grant of the patent: **23.12.2015**
(21) Application number: 09745255.1
(22) Date of filing: 30.10.2009
(51) Int. Cl.: A61L 29/08, A61L 29/14, A61L 29/18

(54) **MEDICAL DEVICES WITH DUAL MODALITY VISIBLE SEGMENTS**
MEDIZINISCHE VORRICHTUNGEN MIT SICHTBAREN DOPPELMODALITÄTSSEGMENTEN
DISPOSITIFS MÉDICAUX COMPORTANT DES SEGMENTS VISIBLES AU MOYEN DE DEUX MODALITÉS

(30) Priority: 23.11.2008 US 117200 P
(43) Date of publication of application: 12.10.2011
(73) Proprietor: Medtronic, Inc, Minneapolis, MN 55432 (US)
(72) Inventor: EBERT, Michael, J., Fridley MN 55421 (US); WALDHAUSER, Steven, L., White Bear Township MN 55110 (US); SCHNEIDER, Mark, D., Mound MN 55364 (US)
(74) Representative: Golding, Louise Ann
(86) International application number: PCT/US2009/062775
(87) International publication number: WO 2010/059409

(56) References cited:
- WO-A2-2005/035043
- WO-A2-2010/059408
- US-A1- 2006 074 401

## Description

### TECHNICAL FIELD

The present disclosure relates generally to medical devices and, more particularly, to medical devices that include selected segments that are visible using both fluoroscopy and ultrasonic imaging.

### BACKGROUND

It may be desirable to monitor the position of medical devices such as, e.g., catheters, while they are within a patient's body. For example, it may be useful to monitor the position of guide catheters which are used to place catheters, electrode leads and the like in desired locations within the body of a patient. A guide catheter typically includes an elongated sheath that is inserted into a blood vessel or another portion of the body. A catheter or lead is introduced through an inner channel defined by the sheath.

To enable precise positioning of a medical device, one or more selected segments may include visibility materials that are visible under fluoroscopy and/or ultrasonic imaging. Using fluoroscopic or ultrasonic imaging techniques, the physician can visualize the guide catheter, and place the catheter or electrode lead in a desired position. Guide catheters, for example, may incorporate radiopaque and/or echogenic materials to promote visibility. Examples of such devices are disclosed in US 2006/074401 and WO 2005/035043.

### BRIEF DESCRIPTION OF THE VIEWS OF THE DRAWING

The present disclosure will be further described with reference to the figures of the drawing, wherein:
FIG. 1 is a perspective view of one exemplary medical device in the form of a catheter.
FIG. 2 is an enlarged cross-sectional view of a portion of one embodiment of a catheter according to the present disclosure.
FIG.3 is an enlarged cross-sectional view of a portion of another embodiment of a catheter according to the present disclosure.

### DETAILED DESCRIPTION OF EXEMPLARY EMBODIMENTS

The present disclosure is directed to medical devices (such as, e.g., catheters, guide catheters, etc.) that include one or more selected segments that are constructed using visibility materials compounded with one or more polymeric materials that make the selected segments visible using both fluoroscopy and ultrasonic imaging. The visibility material takes the form of tungsten and/or tungsten carbide particles dispersed within a polymeric material comprising a polyether block amide.

The use of visibility material as described herein can potentially promote enhanced visibility of a medical device (such as a guide catheter) using both fluoroscopic and ultrasonic imaging techniques. This feature provides a physician with flexible imaging options. The medical device can be used by physicians who prefer fluoroscopic imaging, for example, as well as those who prefer ultrasound. In each case, the physician may select the same guide catheter without regard to the desired imaging modality.

In some embodiments, the use of a visibility material that is both echogenic and radiopaque as an additive to enable both fluoroscopic and ultrasonic imaging can potentially allow the overall additive level to be lower, which may tend to preserve the mechanical properties of a composite blend incorporating the visibility material.

In some instances, a reduced amount of visibility material in a composite blend may also enhance the slittability of a guide catheter. In this manner, the visible material can potentially be loaded into polymeric material in amounts that promote visibility while maintaining slittability.

Although it is known that polyether block amides can be loaded with tungsten and/or tungsten carbide to provide visibility using both fluoroscopy and ultrasonic imaging, these compositions can, in some instances, suffer from degradation during aging. The degradation can adversely impact the flexibility and other mechanical properties of the segments in which the visibility materials are provided. The approaches to providing the visible medical device segments as described herein may address these aging issues while still retaining an suitable level or enhanced level of visibility using both fluoroscopic and ultrasonic imaging.

In one embodiment, the present disclosure provides a medical device. The medical device includes: an inner layer including a radiopaque and echogenic material, wherein the radiopaque and echogenic material includes tungsten and/or tungsten carbide particles distributed within a base polymeric material (i.e., a first polymeric material) including a polyether block amide; and an outer layer including an additional polymeric material (i.e., a second polymeric material). The additional polymeric material is a polyurethane which is more resistant to hydrolysis and/or oxidation than the base polymeric material. In certain embodiments, the medical device is a guide catheter including an elongated sheath including a proximal end and a distal tip, and at least a portion of the distal tip and/or elongated sheath includes the radiopaque and echogenic material. Methods of preparing such medical devices are also provided.

In another embodiment, the present disclosure provides a method of increasing the shelf life of at least a portion of a medical device. In certain embodiments, the method includes: providing a radiopaque and echogenic material including tungsten and/or tungsten carbide particles distributed within a base polymeric material (i.e., a first polymeric material) comprising a polyether block amide; and forming a layer including an additional polymeric material (i.e., a second polymeric material) which is a polyurethane over the radiopaque and echogenic material, wherein the additional polymeric material is more resistant to hydrolysis and/or oxidation than the base polymeric material.

The above brief description is not intended to describe each embodiment or every implementation of the present disclosure. Rather, a more complete understanding of the disclosure will become apparent and appreciated by reference to the following description and claims in view of the accompanying figures of the drawing.

In the following detailed description of illustrative embodiments of the disclosure, reference is made to the accompanying figures of the drawing which form a part hereof, and in which are shown, by way of illustration, specific embodiments that may be practiced. It is to be understood that other embodiments may be utilized and structural changes may be made without departing from the scope of the present disclosure.

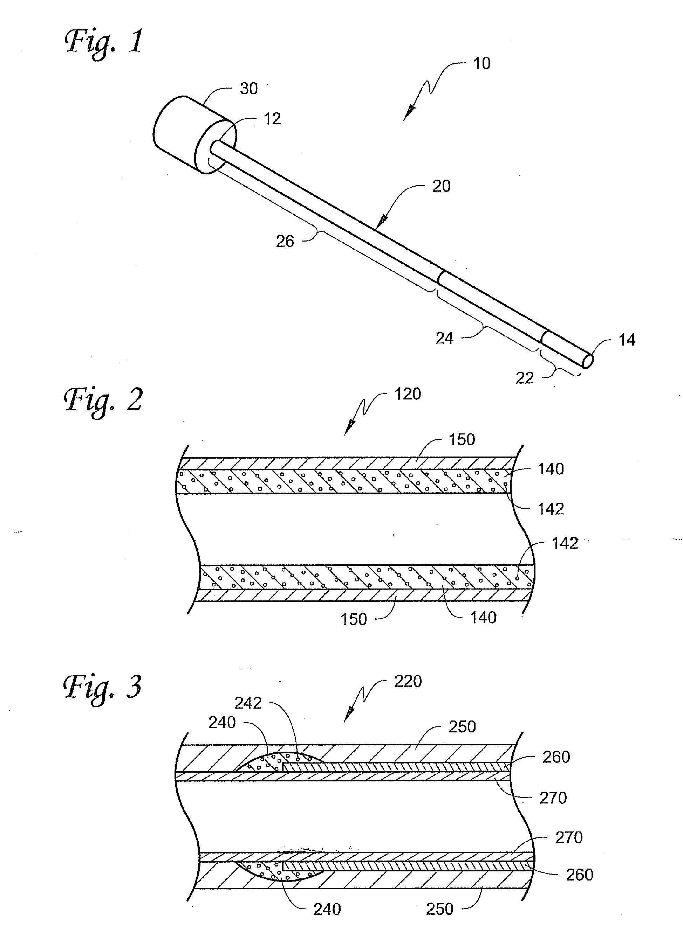

FIG. 1 is a perspective view of one medical device that may be used in connection with the present disclosure which is in the form of a catheter 10. As shown in FIG. 1, catheter 10 includes a proximal end 12, distal tip 14, and an elongated sheath 20 extending between the proximal and distal ends. Catheter 10 may be sized for insertion into a lumen, such as a blood vessel, within the human body. Catheter 10 preferably defines at least one inner channel (not shown in FIG. 1) through which other elements such as other catheters, electrode leads, etc. may be inserted. A handle 30 may be coupled to proximal end 12 of catheter 10. A slitter (not shown) may be positioned near proximal end 12, e.g., adjacent handle 20 if the catheter 10 is to be slit during removal of the catheter 10.

The sheath 20 of the catheter 10 may include any number of segments, with the sheath 10 including three segments 22, 24, and 26 disposed along the length of catheter 10. Sheath 20 may be formed to provide either a straight or pre-bent shape to catheter 10, depending on the desired end application.

Sheath 20 may be manufactured using materials that promote maneuverability and that may also permit slitting along the length of catheter 10. In particular, each segment 22, 24, and 26 of the sheath 20 may be constructed of one or more polymeric materials, such as polyether block amide, nylon block polymer, silicone, or polyurethane, as well as composites or mono-polymers. An example of one potentially suitable group of polymeric materials that can be used are polyether block amides marketed under the trademark PEBAX and commercially available from Arkema, Inc. (Philadelphia, PA).

In some embodiments, the sheath may also include other components such as, e.g., reinforcing braids, etc. If a reinforcing braid is provided, it may terminate short of the distal segment 22 such that the flexibility of the sheath 16 is higher within at least the distal segment 22 as compared to the portions of the sheath 16 that do include a reinforcing braid. Examples of some sheath constructions including reinforcing braids or strands are described in, e.g., U.S. Patent No. 7,065,394 (Hobot et al.).

Medical devices of the present disclosure, such as catheter 10, are constructed to exhibit properties that enhance visibility of selected portions of the medical device when using fluoroscopic or ultrasonic imaging techniques. With reference to FIG. 1, distal segment 22 is found at the distal tip of catheter 10 and incorporates visibility material that makes the segment 22 visible using both fluoroscopy and ultrasonic imaging modalities. The visibility material also may be provided in other segments and/or along the entire length of the catheter.

Each sheath segment 22, 24, and 26 may, in some embodiments, be constructed from a similar material with a similar concentration of tungsten and/or tungsten carbide particles. However, the different sheath segments may be manufactured with polymeric materials that have different hardness characteristics. It may be preferred that the distal segment 22 be flexible such that the distal end of the catheter 10 is more flexible than the segments 24 and 26 such that that tip 14 of the catheter 10 is relatively atraumatic. As a particular illustration, sheath segments 22, 24, and 26 may be constructed from PEBAX material with 25, 35, and 55 Shore D hardnesses, respectively. In another illustration, each sheath segment 22, 24, and 26 may be independently constructed from PEBAX material having a desired hardness (e.g., 25, 35, 40, 55, 63, 70, or 72 Shore D hardness). The tungsten and/or tungsten carbide particles can be added to selected sheath segments 22, 24, and 26 in concentrations that do not significantly degrade the overall mechanical properties of sheath 20, e.g., on the order of about 60 to about 90 percent by weight. As previously described, other ranges can be used. In another embodiment, the tungsten and/or tungsten carbide particles can be added to selected sheath segments 22, 24, and 26 in concentrations on the order of about 60 to about 85 percent by weight. In still yet another embodiment, the tungsten and/or tungsten carbide particles can be added to selected sheath segments 22, 24, and 26 in concentrations on the order of about 60 to about 80 percent by weight. In another embodiment, the tungsten and/or tungsten carbide particles can be added to selected sheath segments 22, 24, and 26 in concentrations on the order of about 65 to about 80 percent by weight. In another embodiment, the tungsten and/or tungsten carbide particles can be added to selected sheath segments 22, 24, and 26 in concentrations on the order of about 65 to about 75 percent by weight.

As one particular example, the tungsten and/or tungsten carbide particles may be added to the base polymeric material (also referred to as the first polymeric material) in sheath segment 22 in the amount of about 70 to about 75 percent by weight and, more preferably, about 73 to about 74 percent by weight. In an exemplary embodiment, the jet milled tungsten carbide material is added to the polymeric material in a weight of about 73.2 percent by weight. A concentration of 73.2 percent by weight tungsten carbide particles to PEBAX material corresponds to a concentration of about 15 percent by volume. Barium sulfate particles may be added to sheath segments 24 and 26 in the amount of about 20 to about 40 percent by weight and, more preferably, about 30 percent by weight.

If the catheter 10 is a guide catheter that is to be slit along its length during use, the addition of tungsten and/or tungsten carbide particles may offer exceptional echogenicity and, when added to the polymeric material, permit ready slitting along the length of catheter 10. As a result, it may be desirable to balance the degree of echogenicity against the slittability of sheath 20. As more tungsten and/or tungsten carbide particles are added to segments 22, 24, and 26, the material forming the sheath 20 becomes difficult to process and, in some cases, difficult to maneuver for insertion into and removal from the body of a patient.

Although polyether block amides can be loaded with tungsten and/or tungsten carbide particles to provide visibility using both fluoroscopy and ultrasonic imaging, it has been discovered that, in some instances, these compositions can potentially suffer from degradation due to temperature (e.g., oxidation) and/or humidity (e.g., hydrolysis). The degradation can adversely impact the flexibility and other properties of the segments in which the visibility materials are provided.

It has been found that by using an outer layer of an additional polymeric material as herein defined (also referred to as the second polymeric material) that is more resistant to hydrolysis and/or oxidation than the base polymeric material, over a visibility material-loaded inner layer (i.e., a tungsten and/or tungsten carbide loaded polyether block amide core), the desired visibility can be attained, and in preferred embodiments, the degradation due to temperature and/or humidity can potentially be reduced.

In certain embodiments, the softening temperature (e.g., melt temperature or glass transition temperature) of the additional polymeric material can be lower than the softening temperature of the base polymeric material. In certain embodiments, the softening temperature (e.g., melt temperature or glass transition temperature) of the additional polymeric material can be the same as or higher than the softening temperature of the base polymeric material.

FIG.2 is an enlarged cross-sectional view of a sheath 120 to illustrate one such construction in which an inner visibility layer 140 that includes particles 142 in a base polymeric material (i.e., polyether block amide) is jacketed by an outer layer 150 that is constructed of an additional polymeric material. In some embodiments, the outer layer 150 may extend only over limited portions of the length of the sheath, for example, over only those portions of the sheath 120 that include the particulate loaded inner visibility layer 140 for which protection is desired.

In some medical devices (e.g., catheters), it can be desirable to use a high visibility band in the form of a visibility material loaded into a polymeric ring that is located between a distal segment and the proximal segment of the device. The high visibility band may preferably include a highly loaded tungsten and/or tungsten carbide particles in a polymeric carrier. The band (i.e., that forms the inner layer) can then be overlaid with an additional polymeric material (i.e., that forms the outer layer) loaded, for example, with BaSO₄, with the additional polymeric material extending over the high visibility band and extending over the distal segment to the distal tip of the device.

Referring to FIG. 3, the polymeric carrier forming the band 240 may preferably be selected for its ability to be highly loaded with tungsten and/or tungsten carbide particles while retaining some level of flexibility. An example of one potentially suitable group of polymeric materials that can be used for the ring 240 are polyether block amides marketed under the trademark PEBAX. It may further be preferred that the polymeric materials of the ring 240 also remain slittable if the medical device is a guide catheter that is to be slit during use.

One embodiment of such a construction is depicted in FIG. 3 which is an enlarged cross-sectional view of a portion of one sheath having an inner sleeve 270 over which an optional braided layer 260 is positioned. The sheath 220 also includes a band 240 that is highly loaded with tungsten and/or tungsten carbide particles 242. The band 240 may preferably be positioned over the end of the braided layer 260 to, e.g., assist in containing the filaments or wires making up the braided layer in addition to providing visibility. All of the features, such as the band 240, braided layer 260, and inner sleeve 270 are then jacketed with an outer layer 250 in a manner similar to that described in connection with FIG. 2.

Medical devices that include a more hydrolytically stable outer layer over a visibility material-loaded inner layer or ring can be prepared by a wide variety of methods including, for example, coextrusion. For example, an inner layer including tungsten and/or tungsten carbide particles in polyether block amide and an outer layer including a polyurethane can be coextruded. Alternatively, the inner layer can be extruded, and the outer layer can subsequently be extruded on the inner layer.

For another example, the band that includes tungsten and/or tungsten carbide particles in the base polymer (i.e., polyether block amide, either in a ring construction or throughout the layer) can be constructed by a wide variety of convenient methods. An outer layer can then be formed over the inner components by a wide variety of convenient methods. Methods for constructing or forming the inner and outer layers include, but are not limited to, forming one or both layers on a mandrel; extruding one or both layers; injection molding one or both layers; compression molding one or both layers, dip coating, fusing, or combinations thereof.

Accordingly, methods of increasing the shelf life of at least a portion of a medical device are also provided herein. For example, an outer layer that is more resistant to hydrolysis and/or oxidation can be provided over a visibility material-loaded inner layer (i.e., a tungsten and/or tungsten carbide loaded polyether block amide core) to form at least a portion of a medical device. In preferred embodiments, a medical device that includes an outer layer that is more resistant to hydrolysis and/or oxidation can have increased shelf life in comparison to comparable medical devices without the outer layer. The outer layer includes the thermoplastic elastomer polyurethane.

ALTERNATIVE/ADDITIONAL APPROACHES: In addition, one or more of the following approaches may be used as an alternative to the approaches described above or in conjunction with the approaches described above to further increase the shelf life of the medical device.

Tungsten particles (as opposed to tungsten carbide particles) can be selected as a visibility material. Generally, tungsten particles may have less of a tendency to effect degradation (e.g., less catalytic filler) than tungsten carbide particles.

Tungsten carbide and/or tungsten particles can be used as the visibility material in a higher durometer polyether block amide (e.g., such as those available under the trade designation PEBAX MX 1205 polyether block amides from Arkema), with the loading level of the visibility material reduced to offset the higher durometer of the polymeric material such that the resulting composite material retains selected mechanical properties, e.g., still provides an atraumatic tip.

One or more additives can be used to enhance stability by reducing hydrolysis and/or oxidation. The additives may include one or more of: antioxidants (e.g., a peroxide decomposer such as IRGAFOS 168; a non-polar anti-oxidant such as IRGANOX 1098; metal deactivators such as IRGANOX MD-1024; etc.); anti-hydrolysis agents (e.g., carbodiimides such as, e.g., CARBAXOL, etc.); etc.

The medical device can be sterilized using ethylene oxide or other sterilization methodologies, and the sterile pouch can be placed into a bag that is impervious to moisture and/or oxygen. Optionally, this bag can be back filled with a dry (inert) gas, such as nitrogen, argon, and the like, and then the bag can be sealed. In another embodiment, the medical device pouch can be placed into a bag that is impervious to moisture and/or oxygen, a dessicant can be added to adsorb any moisture present in the bag, and then the bag can be sealed. For example, the medical device can be sterilized and placed in a moisture proof package that includes added dessicant, the package can be purged with a dry (inert) gas, and the package sealed. For embodiments in which at least a portion of the medical device includes a hydrophilic polymer, the bag can be purged with a humidified gas such that the relative humidity is less than or equal to about 30 percent to prevent unnecessary drying of the hydrophilic polymer.

Illustrative embodiments of this disclosure are discussed and reference has been made to possible variations within the scope of this disclosure. These and other variations and modifications in the disclosure will be apparent without departing from the scope of the disclosure, and it should be understood that this disclosure is not limited to the illustrative embodiments set forth herein.

## Claims

1. A medical device comprising:
an inner layer comprising a radiopaque and echogenic material, wherein the radiopaque and echogenic material comprises tungsten and/or tungsten carbide particles distributed within a base polymeric material comprising a polyether block amide; and
an outer layer comprising an additional polymeric material which is a polyurethane.

2. The medical device of claim 1 wherein the tungsten and/or tungsten carbide particles have an average diameter of less than 1000 nanometers.

3. The medical device of claim 2 wherein the outer layer has a thickness of 5 micrometers to 1000 micrometers.

4. The medical device of claim 1 wherein the tungsten and/or tungsten carbide particles comprise 60 percent by weight to 90 percent by weight of the inner layer.

5. The medical device of claim 1 wherein the medical device is a guide catheter comprising an elongated sheath comprising a proximal end and a distal tip, and at least a portion of the distal tip and/or elongated sheath comprises the radiopaque and echogenic material.

6. A method of preparing at least a portion of a medical device, the method comprising coextruding:
an inner layer comprising a radiopaque and echogenic material, wherein the radiopaque and echogenic material comprises tungsten and/or tungsten carbide particles distributed within a base polymeric material comprising a polyether block amide; and
an outer layer comprising an additional polymeric material which is a polyurethane.

7. The method of claim 6 wherein the medical device is a guide catheter comprising an elongated sheath comprising a proximal end and a distal tip, and at least a portion of the distal tip and/or elongated sheath comprises the radiopaque and echogenic material.

8. A method of preparing at least a portion of a medical device, the method comprising:
providing a radiopaque and echogenic material comprising tungsten and/or tungsten carbide particles distributed within a base polymeric material comprising a polyether block amide; and
forming a layer comprising an additional polymeric material which is a polyurethane over the radiopaque and echogenic material.

9. The method of claim 8 wherein the medical device is a guide catheter comprising an elongated sheath comprising a proximal end and a distal tip, and at least a portion of the distal tip and/or elongated sheath comprises the radiopaque and echogenic material.

10. The method of any one of claims 6 to 9 wherein the radiopaque and echogenic material further comprises one or more antioxidants and/or anti-hydrolysis agents.

## Patentansprüche

1. Medizinische Vorrichtung, die enthält:
eine innere Schicht, die ein röntgenstrahlenundurchlässiges und echogenes Material enthält, wobei das röntgenstrahlenundurchlässige und echogene Material Wolfram- und/oder Wolramkarbidteilchen enthält, die innerhalb eines polymeren Basismaterials, das ein Polyetherblockamid enthält, verteilt sind; und
eine äußere Schicht, die ein zusätzliches polymeres Material enthält, das ein Polyurethan ist.

2. Medizinische Vorrichtung nach Anspruch 1, wobei die Wolfram- und/oder Wolframkarbidteilchen einen mittleren Durchmesser von weniger als 1000 Nanometer haben.

3. Medizinische Vorrichtung nach Anspruch 2, wobei die äußere Schicht eine Dicke im Bereich von 5 Mikrometer bis 1000 Mikrometer aufweist.

4. Medizinische Vorrichtung nach Anspruch 1, wobei die Wolfram- und/oder Wolframkarbidteilchen 60 Gewichtsprozent bis 90 Gewichtsprozent der inneren Schicht ausmachen.

5. Medizinische Vorrichtung nach Anspruch 1, wobei die medizinische Vorrichtung ein Führungskatheter ist, der eine langgestreckte Hülle enthält, die ein proximales Ende und eine distale Spitze enthält, und zumindest ein Teil der distalen Spitze und/oder der langgestreckten Hülle das röntgenstrahlenundurchlässige und echogene Material enthält.

6. Verfahren zum Herstellen zumindest eines Teils einer medizinischen Vorrichtung, wobei das Verfahren umfasst:
Zusammenpressen einer inneren Schicht, die ein röntgenstrahlenundurchlässiges und echogenes Material enthält, wobei das röntgenstrahlenundurchlässige und echogene Material Wolfram- und/oder Wolramkarbidteilchen enthält, die innerhalb eines polymeren Basismaterials, das ein Polyetherblockamid enthält, verteilt sind; und
einer äußeren Schicht, die ein zusätzliches polymeres Material enthält, das ein Polyurethan ist.

7. Verfahren nach Anspruch 6, wobei die medizinische Vorrichtung ein Führungskatheter ist, der eine langgestreckte Hülle enthält, die ein proximales Ende und eine distale Spitze enthält, und zumindest ein Teil der distalen Spitze und/oder der langgestreckten Hülle das röntgenstrahlenundurchlässige und echogene Material enthält.

8. Verfahren zum Herstellen zumindest eines Teils einer medizinischen Vorrichtung, wobei das Verfahren umfasst:
Herstellen eines röntgenstrahlenundurchlässigen und echogenen Materials, das Wolfram- und/oder Wolramkarbidteilchen enthält, die innerhalb eines polymeren Basismaterials, das ein Polyetherblockamid enthält, verteilt sind; und
Bilden einer äußeren Schicht, die ein zusätzliches polymeres Material enthält, das ein Polyurethan ist, über dem röntgenstrahlenundurchlässigen und echogenen Material.

9. Verfahren nach Anspruch 8, wobei die medizinische Vorrichtung ein Führungskatheter ist, der eine langgestreckte Hülle enthält, die ein proximales Ende und eine distale Spitze enthält, und zumindest ein Teil der distalen Spitze und/oder der langgestreckten Hülle das röntgenstrahlenundurchlässige und echogene Material enthält.

10. Verfahren nach einem der Ansprüche 6 bis 9, wobei das röntgenstrahlenundurchlässige und echogene Material ferner ein oder mehrere Antioxidationsmittel und/oder Antihydrolysewirkstoffe enthält.

## Revendications

1. Dispositif médical comportant :
une couche intérieure comportant une matière radio-opaque et échogène, dans lequel la matière radio-opaque et échogène comporte des particules de tungstène et/ou de carbure de tungstène réparties à l'intérieur d'une matière polymère de base comportant un amide de polyéther séquencé ; et
une couche extérieure comportant une matière polymère supplémentaire qui est un polyuréthane.

2. Dispositif médical selon la revendication 1, dans lequel les particules de tungstène et/ou de carbure de tungstène ont un diamètre moyen inférieur à 1 000 nanomètres.

3. Dispositif médical selon la revendication 2, dans lequel la couche extérieure a une épaisseur de 5 micromètres à 1 000 micromètres.

4. Dispositif médical selon la revendication 1, dans lequel les particules de tungstène et/ou de carbure de tungstène comportent 60 pour-cent en poids à 90 pour-cent en poids de la couche intérieure.

5. Dispositif médical selon la revendication 1, dans lequel le dispositif médical est un cathéter-guide comportant une gaine allongée comportant une extrémité proximale et un embout distal, et au moins une partie de l'embout distal et/ou de la gaine allongée comporte la matière radio-opaque et échogène.

6. Procédé de préparation d'au moins une partie d'un dispositif médical, le procédé comportant la coextrusion :
d'une couche intérieure comportant une matière radio-opaque et échogène, dans lequel la matière radio-opaque et échogène comporte des particules de tungstène et/ou de carbure de tungstène réparties à l'intérieur d'une matière polymère de base comportant un amide de polyéther séquencé ; et
d'une couche extérieure comportant une matière polymère supplémentaire qui est un polyuréthane.

7. Procédé selon la revendication 6, dans lequel le dispositif médical est un cathéter-guide comportant une gaine allongée comportant une extrémité proximale et un embout distal, et au moins une partie de l'embout distal et/ou de la gaine allongée comporte la matière radio-opaque et échogène.

8. Procédé de préparation d'au moins une partie d'un dispositif médical, le procédé comportant les étapes consistant à :
fournir une matière radio-opaque et échogène comportant des particules de tungstène et/ou de carbure de tungstène réparties à l'intérieur d'une matière polymère de base comportant un amide de polyéther séquencé ; et
former une couche comportant une matière polymère supplémentaire qui est un polyuréthane au-dessus de la matière radio-opaque et échogène.

9. Procédé selon la revendication 8, dans lequel le dispositif médical est un cathéter-guide comportant une gaine allongée comportant une extrémité proximale et un embout distal, et au moins une partie de l'embout distal et/ou de la gaine allongée comporte la matière radio-opaque et échogène.

10. Procédé selon l'une quelconque des revendications 6 à 9, dans lequel la matière radio-opaque et échogène comporte en outre un ou plusieurs antioxydants et/ou agents anti-hydrolyse.
